(19) Europäisches Patentamt
European Patent Office
Office européen des brevets

(11) **EP 3 597 753 A1**

(12) **EUROPEAN PATENT APPLICATION**
published in accordance with Art. 153(4) EPC

(43) Date of publication:
**22.01.2020 Bulletin 2020/04**

(21) Application number: **18767937.8**

(22) Date of filing: **09.03.2018**

(51) Int Cl.:
*C12N 15/56* (2006.01)   *C08J 11/18* (2006.01)
*C12N 9/16* (2006.01)   *C12P 7/04* (2006.01)
*C12P 7/44* (2006.01)

(86) International application number:
**PCT/JP2018/009170**

(87) International publication number:
**WO 2018/168679 (20.09.2018 Gazette 2018/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.03.2017   JP 2017049198**

(71) Applicant: **Keio University**
**Tokyo 108-8345 (JP)**

(72) Inventors:
• **MIYAMOTO Kenji**
**Yokohama-shi**
**Kanagawa 223-8522 (JP)**

• **FURUKAWA Makoto**
**Yokohama-shi**
**Kanagawa 223-8522 (JP)**
• **KAWAKAMI Norifumi**
**Yokohama-shi**
**Kanagawa 223-8522 (JP)**
• **ODA Kohei**
**Kyoto-shi**
**Kyoto 606-8585 (JP)**

(74) Representative: **J A Kemp LLP**
**14 South Square**
**Gray's Inn**
**London WC1R 5JJ (GB)**

(54) **METHOD FOR IMPROVING ACTIVITY OF PET-DEGRADING ENZYME USING ADDITIVE**

(57)     Provided are a method and a reagent for improving the activity of PETase.

A method for improving PETase activity, comprising adding a surfactant, when the following PETase (a) or (b) is allowed to act on PET so as to degrade the PET:
(a) an aromatic polyester-degrading enzyme consisting of the amino acid sequence as set forth in SEQ ID NO: 2 or 4 in the sequence listing; or
(b) an aromatic polyester-degrading enzyme consisting of an amino acid sequence comprising a deletion, substitution or addition of one or several amino acids with respect to the amino acid sequence as set forth in SEQ ID NO: 2 or 4 in the sequence listing, and having an aromatic polyester-degrading activity.

EP 3 597 753 A1

**Description**

Technical Field

The present invention relates to a technique relevant to degradation of PET (polyethylene terephthalate) resin by using PET hydrolase (hereinafter referred to as "PETase").

Background Art

Since PET resin has high transparency and excellent strength, it has been widely used for bottles and the like. However, since such stable PET resin has not been degraded in the nature, it has caused garbage problems. Thus, the PET resin has become recycled, and at present, chemical recycling has been widely carried out.

In common chemical recycling involving hydrolysis, PET is treated at a high temperature of 150°C to 250°C with a catalyst and excessive water, so that the PET is depolymerized into terephthalic acid (TPA) and ethylene glycol (EG). As a catalyst for hydrolysis, an acid such as sulfuric acid, or a base such as sodium hydroxide is used. However, the chemical recycling has been problematic in terms of a waste liquid treatment, etc.

It has been reported that, aiming at treating such used PET without giving a load to the environment, PET-degrading bacteria have been searched and as a result, a strain (No. 201-F6 strain) completely degrading PET into carbon dioxide and water has been successfully separated (see Patent Literature 1). Moreover, PETase has been isolated from the No. 201-F6 strain (see Patent Literature 2 and Non Patent Literature 1).

However, when the PETase was used alone, PET degradation progressed, but the degradation efficiency was not sufficient.

Citation List

Patent Literature

Patent Literature 1: JP Patent Publication (Kokai) No. 2008-199957 A
Patent Literature 2: International Publication WO 2015/025861

Non Patent Literature

Non Patent Literature 1: Yoshida et al., Science, Vol. 351, Issue 6278, pp. 1196-1199 (2016)

Summary of Invention

Technical Problem

It is an object of the present invention to provide a method for improving the activity of PETase, and a reagent therefor.

Solution to Problem

PETase had been isolated from the strain (No. 201-F6 strain) completely degrading PET into carbon dioxide and water, but the PET-degrading efficiency of the enzyme had not been sufficient. Hence, the present inventors have conducted intensive studies regarding whether the degrading efficiency of the enzyme can be improved by adding an additive into the enzyme reaction system.

As a result, the present inventors have found that, by adding a surfactant such as SDS into the enzyme reaction system and then allowing PETase to act on PET, the PET-degrading activity of the PETase is drastically improved, in comparison to the case of not adding such a surfactant, thereby completing the present invention.

Specifically the present invention is as follows.

[1] A method for improving PETase activity, comprising adding a surfactant, when the following PETase (a) or (b) is allowed to act on PET so as to degrade the PET:

(a) an aromatic polyester-degrading enzyme consisting of the amino acid sequence as set forth in SEQ ID NO:

2 or 4 in the sequence listing; or

(b) an aromatic polyester-degrading enzyme consisting of an amino acid sequence comprising a deletion, substitution or addition of one or several amino acids with respect to the amino acid sequence as set forth in SEQ ID NO: 2 or 4 in the sequence listing, and having an aromatic polyester-degrading activity.

[2] The method according to the above [1], wherein the surfactant is selected from the group consisting of alkyl sulfate, alkane sulfonate, and polyoxyethylene alkyl phenyl ether.

[3] The method according to the above [2], wherein the surfactant is sodium alkyl sulfate having alkyl containing 5 to 30 carbon atoms, or sodium alkane sulfonate having alkane containing 5 to 30 carbon atoms.

[4] The method according to the above [2], wherein the surfactant is polyoxyethylene octyl phenyl ether or polyoxyethylene nonyl phenyl ether.

[5] The method according to any one of the above [1] to [4], wherein the surfactant is added to the PET, and after a predetermined period of time, the PETase is added thereto.

[6] The method according to any one of the above [1] to [4], wherein the surfactant and the PETase are simultaneously added to the PET.

[7] A reagent composition kit for use in PET degradation, which comprises the following PETase (a) or (b) and a surfactant and improves PETase activity:

(a) an aromatic polyester-degrading enzyme consisting of the amino acid sequence as set forth in SEQ ID NO: 2 or 4 in the sequence listing; or

(b) an aromatic polyester-degrading enzyme consisting of an amino acid sequence comprising a deletion, substitution or addition of one or several amino acids with respect to the amino acid sequence as set forth in SEQ ID NO: 2 or 4 in the sequence listing, and having an aromatic polyester-degrading activity.

[8] The reagent composition kit according to the above [7], wherein the surfactant is selected from the group consisting of alkyl sulfate, alkane sulfonate, and polyoxyethylene alkyl phenyl ether.

[9] The reagent composition kit according to the above [8], wherein the surfactant is sodium alkyl sulfate having alkyl containing 5 to 30 carbon atoms, or sodium alkane sulfonate having alkane containing 5 to 30 carbon atoms.

[10] The reagent composition kit according to the above [8], wherein the surfactant is polyoxyethylene octyl phenyl ether or polyoxyethylene nonyl phenyl ether.

[11] The reagent composition kit according to any one of the above [7] to [10], comprising a mixture of the PETase and the surfactant.

[12] The reagent composition kit according to any one of the above [7] to [10], comprising the PETase and the surfactant, separately.

The present description claims priority from Japanese Patent Application No. 2017-049198; the disclosure of which is hereby incorporated by reference.

Advantageous Effects of Invention

PET is treated with a certain type of surfactant, so that the surfactant is allowed to adhere onto the surface of the PET, and thereafter, PETase is allowed to act on the PET, thereby drastically improving the PET-degrading efficiency.

According to the method of the present invention, it becomes possible to practicalize PET bio-recycling.

Brief Description of Drawings

[Figure 1] Figure 1 is a view showing the structures of surfactants used as additives.

[Figure 2] Figure 2 is a view showing the influence of surfactants on PET-hydrolyzing activity.

[Figure 3] Figure 3 is a view showing a change over time in the amount of a PET degradation product in the presence or absence of SDS.

[Figure 4] Figure 4 is a view showing pNPB-hydrolyzing activity in the presence or absence of SDS.

[Figure 5] Figure 5 is a view showing a change in activity for a pre-incubation time.

[Figure 6] Figure 6 is a view showing a relationship between a film area and an SDS concentration.

[Figure 7] Figure 7 is a view showing a comparison made in terms of the amount of a protein adsorbed on a film by addition of a surfactant.

[Figure 8] Figure 8 is a view showing the improvement of PETase activity by sodium alkyl sulfate.

[Figure 9] Figure 9 is a view showing the improvement of PETase activity by sodium alkane sulfonate.

Description of Embodiments

Hereinafter, the present invention will be described in detail.

The present invention relates to a method for improving the enzyme activity of a PET-degrading enzyme (hereinafter referred to as "PETase") by adding an additive to the enzyme reaction system.

Examples of the PETase used in the present invention may include the following enzymes.

PETase is an enzyme that hydrolyzes aromatic polyester resins such as PET resin. This is an aromatic polyester-degrading enzyme (an enzyme that hydrolyzes polyethylene terephthalate (PET), or a partial structure thereof that is bis(2-hydroxyethyl) terephthalate (BHET)).

The PETase can be isolated from Ideonella sp., No. 201-F6 strain that is a Gram-negative bacillus belonging to genus Ideonella isolated from the soil. Isolation of the microorganisms of genus Ideonella can be carried out by a known method. Ideonella sp. No. 201-F6 strain is described in JP Patent Publication (Kokai) No. 2008-199957 A.

The nucleotide sequence of DNA encoding the PETase is as set forth in SEQ ID NO: 1. The amino acid sequence of this enzyme is as set forth in SEQ ID NO: 2. The nucleotide sequence as set forth in SEQ ID NO: 1 comprises the nucleotide sequence of ENA encoding a signal sequence (wherein the sequence consisting of the nucleotides at positions 1 to 81 is the sequence encoding the signal sequence), and the amino acid sequence as set forth in SEQ ID NO: 2 comprises the signal sequence (wherein the sequence consisting of the amino acids at positions 1 to 27 is the signal sequence). The PETase includes those comprising the signal sequence and those not comprising the signal sequence. In addition, the DNA encoding the PETase includes those comprising the nucleotide sequence encoding the signal sequence and those not comprising the nucleotide sequence encoding the signal sequence. An example of the DNA encoding the PETase that does not comprise the signal sequence may be DNA consisting of the nucleotides at positions 82 to 873 of the nucleotide sequence as set forth in SEQ ID NO: 1.

The PETase used in the present invention can be obtained by culturing the above-described Ideonella sp. No. 201-F6 strain, then allowing the strain to generate it, and then purifying it. Otherwise, the present PETase can also be obtained by transforming host microorganisms with a gene encoding the enzyme of the present invention and then culturing the transformed microorganisms. When the present PETase is produced by the latter method, in order to increase the expression level of the PETase in the host microorganisms, the rate of utilizing a codon is preferably optimized depending on the host microorganisms. Optimization of a codon can be carried out by a known method. For example, when the PETase used in the present invention is produced as a recombinant enzyme using *Escherichia coli* as a host, the nucleotide sequence, in which the codon is optimized, is as set forth in SEQ ID NO: 3. The amino acid sequence of the PETase encoded by the gene is as set forth in SEQ ID NO: 4. The amino acid sequence as set forth in SEQ ID NO: 4 is the same as an amino acid sequence obtained by excluding the signal sequence from the amino acid sequence as set forth in SEQ ID NO: 2.

The PETase may comprise a mutation such as a deletion, substitution or addition of at least one, and preferably, one or several amino acids with respect to the aforementioned amino acid sequence, as long as a protein consisting of the amino acid sequence has PETase activity.

For example, at least one, and preferably, one or several (e.g., 1 to 10, preferably 1 to 5, more preferably 1 to 3, and particularly preferably 1 or 2) amino acids may be deleted from the amino acid sequence as set forth in SEQ ID NO: 2 or 4; at least one, and preferably, one or several (e.g., 1 to 10, preferably 1 to 5, more preferably 1 to 3, and particularly preferably 1 or 2) amino acids may be added to the amino acid sequence as set forth in SEQ ID NO: 2 or 4; or at least one, and preferably, one or several (e.g., 1 to 10, preferably 1 to 5, more preferably 1 to 3, and particularly preferably 1 or 2) amino acids of the amino acid sequence as set forth in SEQ ID NO: 2 or 4 may be substituted with other amino acids.

Such an amino acid sequence comprising a deletion, substitution or addition of one or several amino acids with respect to the amino acid sequence as set forth in SEQ ID NO: 2 or 4 may be, for example, an amino acid sequence having a sequence identity of at least 85% or more, preferably 90% or more, more preferably 95% or more, and particularly preferably 97% or more, to the amino acid sequence as set forth in SEQ ID NO: 2 or 4, when the sequence identity is calculated using BLAST (Basic Local Alignment Search Tool at the National Center for Biological Information), etc. (e.g. default, namely, initial setting parameters).

A protein having an amino acid sequence comprising a deletion, substitution or addition of one or several amino acids with respect to the amino acid sequence as set forth in SEQ ID NO: 2 or 4 is substantially identical to a protein having the amino acid sequence as set forth in SEQ ID NO: 2 or 4.

Moreover, DNA, which can hybridize under the below-mentioned stringent conditions with DNA consisting of a sequence complementary to DNA consisting of the nucleotide sequence as set forth in SEQ ID NO: 1 or 3, and encodes a protein having an aromatic polyester-degrading enzyme activity, is included in the DNA of the present invention. That is to say, the stringent conditions mean conditions under which DNA can be identified by performing hybridization using a DNA-immobilized filter in the presence of 0.7 to 1.0 M NaCl at 68°C, and then washing the reaction product at 68°C using 0.1 to 2 x SSC solution (wherein 1 x SSC consists of 150 mM NaCl and 15 mM sodium citrate). Otherwise, this is DNA capable of forming a hybrid by transcribing and immobilizing the DNA on a nitrocellulose membrane according to a Southern blotting method, and then reacting it in a hybridization buffer [50% formamide, 4 x SSC, 50 mM HEPES (pH 7.0), 10 x Denhardt's solution, and 100 μg/ml salmon sperm DNA] at 42°C overnight.

Furthermore, DNA, which has a sequence identity of at least 85% or more, preferably 90% or more, more preferably 95% or more, and particularly preferably 97% or more, to the DNA consisting of the nucleotide sequence as set forth in SEQ ID NO: 1 or 3, when the sequence identity is calculated using BLAST (Basic Local Alignment Search Tool at the National Center for Biological Information), etc. (e.g., default, namely, initial setting parameters), and encodes a protein having PETase activity, is also included in the DNA encoding the PETase.

The PETase used in the present invention can be produced by culturing Ideonella sp. No. 201-F6 strain, and then isolating it from the culture solution of the Ideonella sp. No. 201-F6 strain, etc. according to a known method. In addition, the PETase used in the present invention can be produced as a recombinant enzyme by introducing DNA encoding the PETase into host microorganisms and then culturing the microorganisms. For example, the DNA encoding the PETase is connected with (inserted into) a suitable vector to produce an expression vector, and the expression vector is then introduced into host microorganisms, so that the host microorganisms may be transformed. The vector used to insert the DNA encoding the PETase into the host microorganisms is not particularly limited, as long as it is able to replicate in host cells such as bacteria, yeasts or animal cells. Examples of the vector may include plasmid DNA and phage DNA. As vector DNA used to construct such an expression vector, widely diffused, easily available DNA is used. Examples thereof may include a pET vector, a pQE vector, a pCold vector, and a pUC19 vector. The method of constructing the expression vector is not particularly limited, and the expression vector can be constructed according to an ordinary method. The host cells transformed with the expression vector are not particularly limited, as long as they can express the DNA encoding the PETase. Examples of the bacteria used as such an expression vector may include *Escherichia coli* and *Bacillus subtilis.* An example of the yeast used as an expression vector may be *Saccharomyces cerevisiae.* Examples of the animal cells used as an expression vector may include Chinese hamster ovarian (CHO) cells, monkey COS cells, and mouse fibroblasts.

The PETase generated by host cells can be purified by known purification methods such as, for example, gel filtration chromatography, ultrafiltration, ion exchange chromatography, affinity chromatography, hydrophobic chromatography, chromatofocusing, isoelectric focusing, and gel electrophoresis, which are applied alone or in combination.

The additive that improves the activity of the PETase may be, for example, a compound having a hydrophilic region and a hydrophobic region. The additive that improves the activity of the PETase binds to the PETase in the hydrophilic region thereof, and adheres to the surface of PET in the hydrophobic region thereof. As a result, the amount of the PETase adsorbed on the surface of the PET is increased, and the PETase is contacted with the PET to promote the enzyme reaction, so that the PET-degrading efficiency is improved. A representative compound having a hydrophilic region and a hydrophobic region may be a surfactant.

Examples of the surfactant improving the activity of the PETase may include: anionic surfactants such as monoalkyl sulfate, alkyl polyoxyethylene sulfate, alkyl benzenesulfonate, and monoalkyl phosphate; cationic surfactants such as alkylamine salts; amphoteric surfactants such as alkyl dimethyl amine oxide and alkyl carboxy betaine; and nonionic surfactants such as polyoxyethylene alkyl phenyl ether (alkylphenolic surfactant), polyoxyethylene alkyl ether, alkyl polyglucoside, fatty acid diethanolamide, and alkyl monoglyceryl ether.

Among these surfactants, anionic surfactants such as alkyl sulfate and alkane sulfonate, or alkylphenolic surfactants that are nonionic surfactants are used herein.

Examples of the alkyl sulfate may include sodium salts of long chain alkyl esters of sulfuric acid (sodium alkyl sulfate (sodium alkyl ester sulfate and higher alcohol sodium ester sulfate)). Examples of the alkyl of the sodium alkyl sulfate include alkyl containing 5 to 30 carbon atoms, preferably alkyl containing 10 to 15 carbon atoms, and more preferably alkyl containing 12 to 14 carbon atoms.

Examples of the sodium alkyl sulfate containing 5 to 30 carbon atoms may include sodium pentyl sulfate (C5), sodium hexyl sulfate (C6), sodium heptyl sulfate (C7), sodium octyl sulfate (C8), sodium nonyl sulfate (C9), sodium decyl sulfate (C10), sodium undecyl sulfate (C11), sodium dodecyl sulfate (C12), sodium tridecyl sulfate (C13), sodium tetradecyl sulfate (C14), sodium pentadecyl sulfate (C15), sodium hexadecyl sulfate (C16), sodium heptadecyl sulfate (C17), sodium octadecyl sulfate (C18), sodium nadecyl sulfate (C19), sodium icosyl sulfate (C20), sodium heneicosyl sulfate (C21), sodium docosyl sulfate (C22), sodium tricosyl sulfate (C23), sodium tetracosyl sulfate (C24), sodium pentacosyl sulfate (C25), sodium hexacosyl sulfate (C26), sodium heptacosyl sulfate (C27), sodium octacosyl sulfate (C28), sodium nonacosyl sulfate (C29), and sodium triacontyl sulfate (C30).

The alkane sulfonate includes straight chain sodium alkane-1-sulfate. Examples of the alkane of the sodium alkane sulfonate may include alkane containing 5 to 30 carbon atoms, preferably alkane containing 10 to 15 carbon atoms, and more preferably alkane containing 12 to 14 carbon atoms.

Examples of the sodium alkane sulfonate containing 5 to 30 carbon atoms may include sodium pentane-1-sulfonate (C5), sodium hexane-1-sulfonate (C6), sodium heptane-1-sulfonate (C7), sodium octane-1-sulfonate (C8), sodium nonane-1-sulfonate (C9), sodium decane-1-sulfonate (C10), sodium undecane-1-sulfonate (C11), sodium dodecane-1-sulfonate (C12), sodium tridecane-1-sulfonate (C13), sodium tetradecane-1-sulfonate (C14), sodium pentadecane-1-sulfonate (C15), sodium hexadecane-1-sulfonate (C16), sodium heptadecane-1-sulfonate (C17), sodium octadecane-1-sulfonate (C18), sodium nonadecane-1-sulfonate (C19), sodium icosane-1-sulfonate (C20), sodium heneicosane-1-sulfonate (C21), sodium docosane-1-sulfonate (C22), sodium tricosane-1-sulfonate (C23), sodium tetracosane-1-sulfonate (C24), sodium pentacosane-1-sulfonate (C25), sodium hexacosane-1-sulfonate (C26), sodium heptacosane-1-sulfonate (C27), sodium octacosane-1-sulfonate (C28), sodium nonacosane-1-sulfonate (C29), and sodium triacontane-1-sulfonate (C30).

The alkylphenolic nonionic surfactant includes polyoxyethylene alkyl phenyl ether (alkyl phenol ethoxylate). The polyoxyethylene alkyl phenyl ether has a structure in which a hydrophilic polyoxyethylene (POE) chain binds to a hydrophobic alkylphenol group via an ether bond. Specific examples of the polyoxyethylene alkyl phenyl ether may include polyoxyethylene octyl phenyl ether and polyoxyethylene nonyl phenyl ether. Such alkylphenolic nonionic surfactants are available, in the form of a mixture of a large number of compounds each having a different POE length, as products each having a different mean chain length. Examples of a product of polyoxyethylene octyl phenyl ether may include Triton[(R)] X series (Dow Chemical) such as Triton X-100, Triton X-114, and Triton X-405. Examples of a product of polyoxyethylene nonyl phenyl ether may include Tergitol[(R)] NP series (Dow Chemical) and Igepal[(R)] CO series (Rhodia).

When PET is degraded by using the PETase, the shape of the PET to be degraded is not limited, and examples of the shape of the PET may include fibrous, particulate, flake-like, pellet-like, film-like, massive, and bottle-like shapes. In addition, a mixture thereof can also be used.

The PETase used in the present invention can degrade isophthalic acid copolymeric amorphous PET (iso PET) and terephthalic acid copolymeric amorphous PET (tere PET). The present PETase can degrade, for example, an iso PET film and a tere PET film, which are placed on a film. The present PETase can degrade iso PET and tere PET at a rate of, for example, 0.02 mg/cm$^2$/day or more, preferably 0.05 mg/cm$^2$/day or more, more preferably 0.1 mg/cm$^2$/day or more, and also, at a rate of, for example, 0.3 mg/cm$^2$/day or less, preferably 0.5 mg/cm$^2$/day or less, more preferably 1.0 mg/cm$^2$/day or less. Whether or not the PET has been degraded by the PETase of the present invention can be determined, for example, by using, as an indicator, generation of monohydroxyethyl terephthalate (TA-EG, MHET) or terephthalic acid (TPA), each of which is a degradation product of PET.

Accordingly, whether or not the above-described additive has improved the activity of PETase may be determined by allowing PET to react with PETase, and then measuring a degradation product of PET. In addition, the activity of PETase can be measured by using a pNPB (p-nitrophenyl-butyrate)-hydrolyzing activity as an indicator.

Degradation of PET by PETase can be carried out by allowing the PETase to act on the PET. When the PETase is allowed to act on the PET, the above-described additive may be added. The term "act" is used herein to mean that PET is allowed to come into contact with an enzyme so as to cause an enzyme reaction.

The method of adding the additive is not limited. For example, the additive may be first added to the PET, and the PETase may be then allowed to act thereon. For example, a solution containing the additive and the PET may be placed into a vessel, followed by treating them for a predetermined period of time, and thereafter, the PETase may be added into the vessel. By adding the additive to the PET and then leaving them for a predetermined period of time, the additive adheres onto the surface of the PET. The additive may be added to the PET and they may be left for several hours to several days, and thereafter, the PETase may be added thereto. During a period from addition of the additive to the PET until addition of the enzyme thereto, they may be left at rest, or may be stirred.

Otherwise, PET, an additive and PETase may be simultaneously added, for example, in the form of a mixed solution, into a vessel, so that the treatment with the additive and the enzyme reaction may be simultaneously carried out. In this case also, they may be left at rest, or may be stirred.

By using a large-scale treatment vessel, a large amount of PET can be degraded at one time.

Further, the additive is allowed to adhere onto the surface of the PET, and thereafter, they are placed into an enzyme solution, so that the enzyme reaction may be carried out.

When the enzyme reaction is carried out by treating the PET with the additive, the reaction may be carried out in a buffer. The used buffer is not limited, and a Tris buffer, a Good's buffer or the like may be used, as appropriate. Since the optimal pH of the PETase is approximately pH 9.0, the pH of the buffer is adjusted to pH 8 to 10, and preferably pH 8.5 to 9.5. The amount of the PET with respect to the amount of the solution is not limited. The amount of the solution and the amount of the PET may be adjusted, so that the PET can be completely immersed in the solution.

When the PET is degraded according to the enzyme reaction, the concentration of the PETase and the amount of the additive may be determined, as appropriate, depending on the amount of the PET to be degraded, etc.

The concentration of the PETase is preferably 10 nM to 5000 nM (0. 28 to 140 μg/mL), and more preferably 20 nM to 1000 nM (0. 56 to 28 μg/mL). In addition, although the concentration of the additive is different depending on the compound used, it is preferably 0.001 to 0.1 (w/v)%, more preferably 0.005 to 0.05 (w/v)%, and particularly preferably 0.01 to 0.05 (w/v)%. When the PET is degraded in a solution, the concentration of the PETase and the concentration of the additive in the solution may be adjusted to the above-described concentrations. On the other hand, when the PET was treated by allowing the additive to adhere onto the surface of the PET, the concentration of the additive to be adhered may be adjusted to the above-described concentration.

Moreover, for example, when the surface area of the PET to be treated can be calculated, 1 mL of the additive in a concentration of 0.01 to 0.05 (w/v)% may be added with respect to 0.1 to 100 cm$^2$ of the surface area of the PET to be degraded. Preferably, 1 mL of the additive in a concentration of 0.01 to 0.05 (w/v)% may be added with respect to 1 to 20 cm$^2$ of the surface area of the PET. Thereafter, the enzyme may be added thereto, in a concentration of preferably 0.001 to 0.1 (w/v)%, more preferably 0.005 to 0.05 (w/v)%, and particularly preferably 0.01 to 0.05 (w/v)%.

Upon degradation of the PET, the reaction temperature is 15°C to 50°C, preferably 20°C to 40°C, and particularly preferably 25°C to 30°C. The pH applied during the reaction is around pH 9.0, preferably pH 8.0 to 10.0, and more preferably pH 8.5 to 9.5. The reaction time may be determined, as appropriate, depending on the amount of the PET to be degraded, and it is several hours to several months. When a long-term treatment is carried out, PETase may be added on a regular basis.

The present invention encompasses a reagent composition for degrading PET. This reagent composition comprises PETase and an additive. The reagent may be a mixture of the PETase and the additive, or may also comprise the PETase and the additive, which are placed in different vessels.

That is to say, the present invention encompasses a PET-degrading reagent composition comprising a mixture of PETase and an additive, or a PET-degrading reagent composition comprising PETase and an additive, separately. The reagent composition is also referred to as a "reagent composition kit."

The concentration of the PETase and the concentration of the additive are not limited. For example, the concentration of the PETase is preferably 10 nM to 5000 nM (0. 28 to 140 μg/mL), and more preferably 20 nM to 1000 nM (0.56 to 28 μg/mL). On the other hand, the concentration of the additive is preferably 0.001 to 0.1 (w/v)%, more preferably 0.005 to 0.05 (w/v)%, and particularly preferably 0.01 to 0.05 (w/v)%. The PETase or the additive may be added into a buffer. The buffer is not limited, and a Tris buffer, a Good's buffer or the like may be used, as appropriate. The pH of the buffer is pH 8 to 10, and preferably pH 8.5 to 9.5.

In the case of using the reagent composition comprising a mixture of the PETase and the additive, PET may be placed in a PET-degrading reagent composition vessel, and the mixture of the PETase and the additive may be added therein, so that they may be reacted with each other. On the other hand, in the case of using the PET-degrading regent kit comprising the PETase and the additive, separately, PET may be added to a PET-degrading reagent composition vessel. Thereafter, the additive may be first added into the vessel, so that the surface of the PET may be treated with the additive, and after that, the PETase may be added thereto to promote a degradation reaction. Otherwise, the additive and the PETase comprised in the kit may be simultaneously added.

With the PETase used in the present invention, wastes such as PET bottles can be treated. PET is degraded with the PETase, and the degradation products can be then used in recycling. Furthermore, using the PETase used in the present invention, modification of the surface of PET processed products such as PET films, modification of the surface of PET fibers, washing of clothes in which PET fibers are used, washing of PET resins subjected to recycling, and the like can be carried out.

Examples

The present invention will be specifically described in the following examples. However, these examples are not intended to limit the scope of the present invention.

[Experimental Examples]

In the following examples, the activity of PETase (PET-hydrolyzing activity) was measured using, as indicators, the amount of a degradation product and pNPB (p-nitrophenyl-butyrate)-hydrolyzing activity. Hereafter, a method of measuring pNPB (p-nitrophenyl-butyrate)-hydrolyzing activity is described.

pNPB used as an artificial substrate in the measurement of esterase activity is hydrolyzed into 1-butanoic acid and p-nitrophenol (pNP). At this time, since pNP exhibits yellow color, the concentration thereof can be measured using an absorption spectrometer.

The pNPB-hydrolyzing activity immediately after purification of the enzyme was measured. The composition of a reaction solution is shown in Table 1. pNPB was dissolved in dimethyl sulfoxide (DMSO). The enzyme was mixed with a buffer, and the obtained mixture was then incubated at 30°C for 30 minutes. Thereafter, the substrate was added to the reaction mixture, and a change in the absorbance at 425 nm for 1 minute was measured three times. The inclination (ΔAbs) of the measured absorbance change was applied to the following equation to obtain catalyst turnover frequency.

$$TOF = \Delta Abs \; x \; (\varepsilon \; x \; c \; x \; l \; x \; \Delta t)^{-1}$$

TOF: Catalyst turnover frequency; $\varepsilon$: Molar absorption coefficient; c: Enzyme concentration; 1: Thickness of solution layer; $\Delta t$: Reaction time.

With regard to the pNPB-hydrolyzing activity, the catalyst turnover frequency became 1.80 g$^{-1}$ when the concentration of the enzyme was 250 nM and the concentration of pNPB was 100 $\mu$M, and thus, it was found that the activity can be measured using pNPB.

[Example 1] Expression and purification of PETase

Expression of PETase

The ORF2645 protein expression vector pET21-b(+) optORF2645 was introduced into the competent cells of *Escherichia coli* BL21 Codon Plus (DE3) RIPL (Agilent) according to a heat shock method. The vector pET21-b(+) optORF2645 was constructed by the method described in International Publication WO2015/025861. Subsequently, the transformed cells were subjected to liquid culture, and were then induced by IPTG, so that the cells were allowed to express a protein. However, when the present enzyme is overexpressed in a cell mass, a majority thereof becomes insoluble. Hence, the acquisition of an active enzyme by refolding was analyzed. First, the insoluble enzyme was solubilized using Urea. Thereafter, a culture supernatant, a homogenized supernatant, and a solubilized solution were subjected to SDS-PAGE. As a result, a dense band of PETase was observed around 28 kDa in the solubilized solution, and thus, it was found that the enzyme could be solubilized.

Purification of solubilized solution according to nickel affinity chromatography

Since the present enzyme was successfully solubilized, the enzyme was then purified according to nickel affinity chromatography. Thereafter, each eluate obtained by purification with a column was subjected to SDS-PAGE. As a result, a band of PETase (approximately 28 kDa) could be confirmed in an eluate fraction.

[Example 2] Improvement of PET-hydrolyzing activity by using surfactant (1) Methods

1. PET-hydrolyzing activity in presence of surfactant

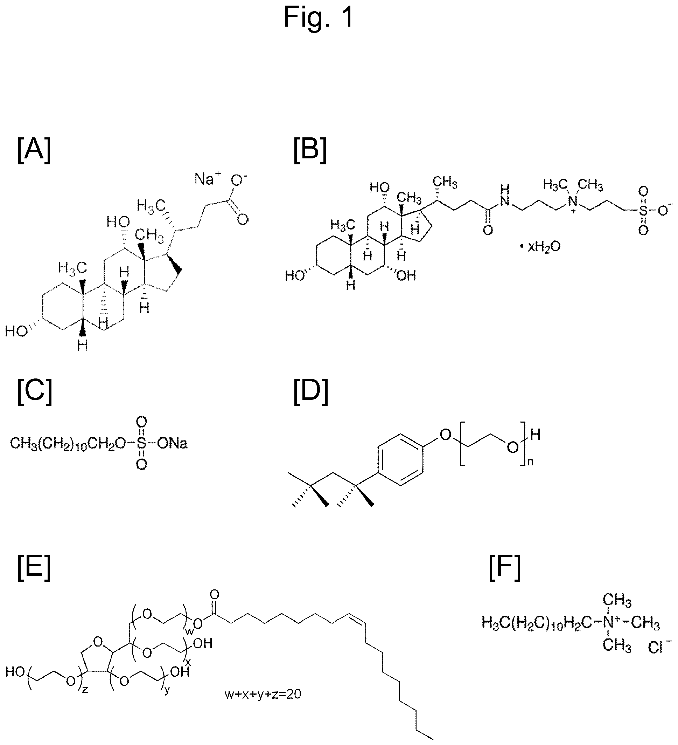

In addition to the optimal conditions, a surfactant was added in a concentration of 0.001 to 0.01 (w/v)%, and the activity was then measured. As surfactants, six types of surfactants, namely, [A] Sodium Cholate (SC), [B] 3-[(3-Cholamidopropyl)dimethylammonio]propanesulfonate (CHAPS), [C] Sodium dodecyl sulfate (SDS), [D] Octylphenol ethoxylate (Triton X-114), [E] Polysorbate 80 (Tween 80), [F] and Dodecyltrimethylammoniumu chloride (DTMA-Cl), were used (Figure 1). The surfactant, a buffer and a film were added to the reaction system, and the obtained mixture was then pre-incubated at 30°C for 1 hour. Thereafter, the concentration of the enzyme was set at 50 nM, the film area was set at 3.39 cm$^2$/mL, and the reaction was carried out for 3 hours.
A system, to which no surfactants were added, was used as a control.

2. Reaction activity in presence of SDS

The film area was changed, and the enzyme concentration was set at 50 nM or 500 nM, and thereafter, the activity was measured under the optimal conditions and in the presence of 0.01(v/v)% SDS. Moreover, a comparison was made regarding the case of setting the enzyme concentration at 50 nM and the amount of the degradation product (monohydroxyethyl terephthalate (MHET)) depending on the reaction time.
The amount of the degradation product was measured by reverse phase high performance liquid chromatography (HPLC) in accordance with the method described in International Publication WO 2015/025861.

3. pNPB-hydrolyzing activity

The p-nitrophenyl-butyrate(pNPB)-hydrolyzing activity was measured in the presence of 0.01 (w/v)% SDS, in addition to the conditions shown in Table 1.

[Table 1]

| Composition of pNPB reaction solution | |
|---|---|
| 10 mM pNPB in DMSO | 10 μL |
| Purified enzyme solution | Final concentration 250 nM |
| 50 mM Bicine(pH9.0) | up to 1000 μL |

4. Activity change depending on pre-incubation time

The pre-incubation time after addition of SDS was set at 0 or 1 hour, the reaction time was set at 1 hour, the enzyme concentration was set at 50 nm, and the film area was set at 3.39 $cm^2$/mL. Thereafter, a comparison was made in terms of the activity under the optimal conditions.

5. Film area dependence of optimal SDS concentration

The enzyme concentration was set at 500 nM, the SDS concentration and the film area were changed, and the activity under the optimal conditions was then measured.

6. Amount of enzyme adsorbed on film

The amount of the enzyme adsorbed on the film was observed under conditions in which the activity was changed by addition of a surfactant. SDS was added to the reaction system to result in a concentration of 0.01 (w/v)% to 0.04 (w/v)%, in addition to conditions of an enzyme concentration of 500 nM, a pre-incubation time of 1 hour, a reaction time of 3 hours, and a film area of 1.13 $cm^2$/mL. After completion of the reaction, the film was removed using tweezers, and was then rinsed with distilled water. The film was placed on the lid of a 1.5-mL tube, and thereafter, 10 μL of an SDS-PAGE sample buffer was added thereto. After that, the resultant was left at rest for 10 minutes, and was then centrifuged. The thus centrifuged solution was subjected to SDS-PAGE. Moreover, in addition to the conditions without addition of surfactants, a comparison was made with conditions including addition of DTMA-Cl that was a cationic surfactant.

Results

1. PET-hydrolyzing activity in presence of surfactant

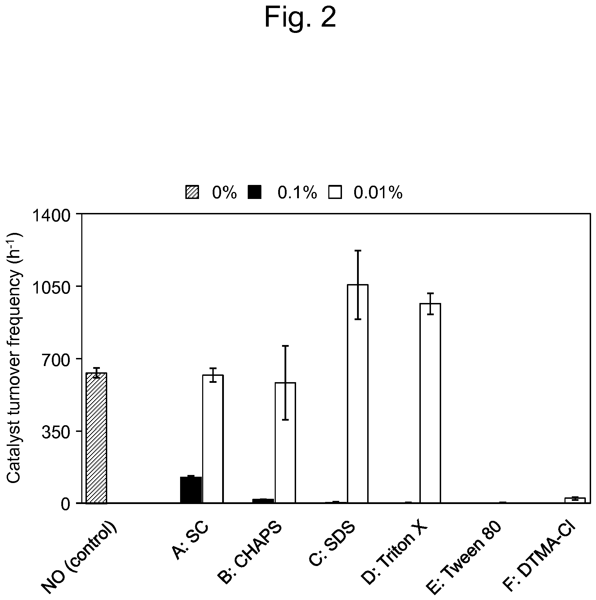

In order to verify the influence of the enzyme activity by addition of a surfactant, the activity was measured under conditions in which a small amount of surfactant was present. As a result, regardless of the type of the surfactant, when the concentration of the surfactant exceeded 0.1% (w/v), the activity was decreased. Thus, when the concentration was decreased to 0.01% (w/v), the activity was found with the surfactants other than Tween 80 and DTMA-Cl (Figure 2). Among others, under conditions including addition of SDS, the activity that was approximately two times greater than the activity of the control was exhibited. However, in the case of DTMA-Cl having a different charge in the hydrophilic region, the activity was significantly decreased.

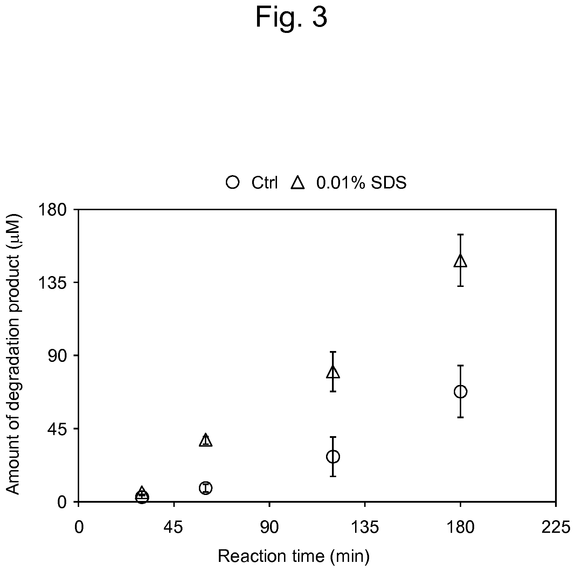

Moreover, it was considered that the surfactant that was a denaturant might have influence on the stability of the enzyme. Hence, a change in the amount of a PET degradation product was measured for a reaction of 3 hours. As a result, it was found that the amount of a PET degradation product was increased in a time-dependent manner. Accordingly, it was found that almost no activity decrease took place at least after the reaction of 3 hours (Figure 3).

How the relationship between the film area and the activity by addition of SDS was changed depending on the enzyme concentration was observed. As a result, when the enzyme concentration was set at 50 nM, the activity became approximately 2.5 times higher than the control under conditions including a film area of 1.13 $cm^2$/mL, and approximately 1.7 times higher than the control under conditions including a film area of 11.3 $cm^2$ /mL (Table 2). Thus, it was found that the change rate of the activity was different depending on the film area, and that the effects were exhibited, in particular, in the case of a small film area.

[Table 2]

| Activity change depending on film area (enzyme concentration: 50 nM) | | | |
|---|---|---|---|
| Film area ($cm^2$/mL) | Control ($h^{-1}$) | Upon addition of 0.01% SDS ($h^{-1}$) | Relative activity (fold) |
| 1.13 | 321 | 793 | 2.47 |
| 2.26 | 453 | 901 | 1.99 |
| 3.39 | 547 | 1020 | 1.86 |
| 5.65 | 726 | 1270 | 1.75 |
| 9.04 | 841 | 1680 | 1.99 |
| 11.3 | 1110 | 1910 | 1.72 |

Furthermore, the activity was measured under conditions of an enzyme concentration of 500 nM in the same manner as described above. As a result, it was found that the activity was exhibited, although almost no activity was exhibited in the case of the control (Table 3). This is considered because the enzyme that became congested on the film could have activity.

From these results, it is suggested that SDS should have influence on the interaction between the film and the enzyme. Thus, in the subsequent experiment, whether or not this change is an action that depends on the interaction between the film and SDS was verified.

[Table 3]

| Activity change depending on film area (enzyme concentration: 500 nM) | | | |
|---|---|---|---|
| Film area ($cm^2$/mL) | Control ($h^{-1}$) | Upon addition of 0.01% SDS ($h^{-1}$) | Relative activity (fold) |
| 1.13 | 0.071 | 154 | 2170 |
| 2.26 | 5.14 | 260 | 50.6 |
| 3.39 | 49.4 | 297 | 6.01 |
| 5.65 | 126 | 451 | 3.58 |
| 9.04 | 199 | 660 | 3.32 |
| 11.3 | 239 | 743 | 3.11 |

2. pNPB-hydrolyzing activity in presence of SDS

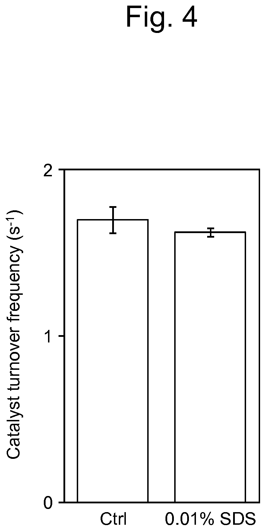

If SDS activated the enzyme, the improvement of the activity should be achieved, regardless of the type of a substrate on which the enzyme is allowed to act. As such, in order to confirm the influence of SDS on the enzyme, the used substrate was converted to pNPB, and the activity was then measured before and after addition of SDS. As a result, the activity was reduced by approximately 5%, which was different from the results of the reaction system with the film (Figure 4). From these results, it was considered that SDS would not improve the enzyme activity, but rather, it would increase the degraded amount by acting on the film.

3. Interaction of PET film with SDS

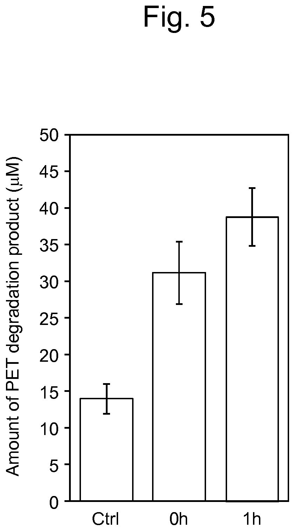

From the results described in the above 2., it was suggested that SDS should act on the film. Hence, the influence of the amount of SDS adsorbed on the film on the activity was verified. It was considered that adsorption of SDS on the film would occur time-dependently, and verification was carried out while changing the pre-incubation time. As a result, the amount of a degradation product was changed depending on the pre-incubation time (Figure 5). From these results, it was found that hydrolysis by the enzyme easily progresses as a result of adsorption of SDS on the film.

In addition, since the degraded amount is increased by adsorption of SDS on the film, it is considered that the optimal SDS concentration changes with the film area as a result of adsorption equilibrium.

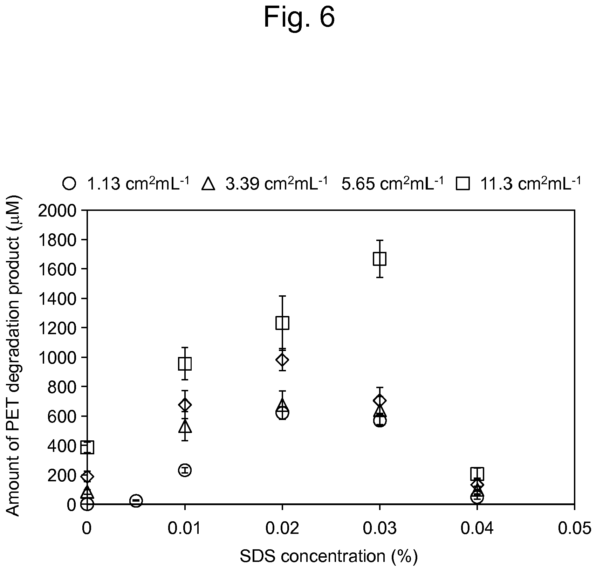

Hence, the influence of the adjustment of the SDS concentration depending on the film area on the activity was examined. As a result, under conditions of a fil area of up to 5.65 cm$^2$/mL, the optimal SDS concentration was 0.02 (w/v)%. In contrast, under conditions of a fil area of up to 11.3 cm$^2$/mL, the optimal SDS concentration was 0.03 (w/v)% (Figure 6). It is considered that this is a change due to the adsorption equilibrium between SDS and the film, and these results suggest that SDS is adsorbed on the film.

From these results, it was found that SDS certainly acts on the film, so as to increase the degraded amount.

4. Amount of enzyme adsorbed on film according to SDS

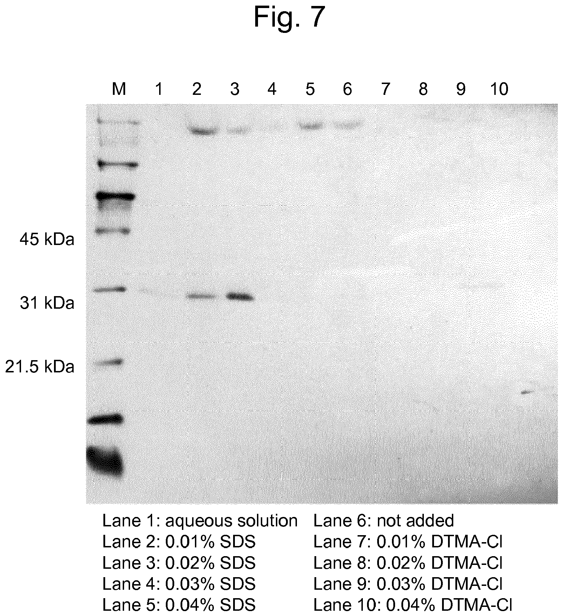

Since SDS interacts with the film to increase the degraded amount, it is considered that efficient degradation is achieved by a change in the amount of the enzyme adsorbed. Hence, the amount of the enzyme adsorbed on the film was confirmed by visualizing it according to SDS-PAGE under conditions including addition of SDS. As a result, adsorption of a large amount of protein was confirmed under conditions including addition of SDS (Figure 7). In view of the foregoing, it was found that SDS increases the amount of the enzyme adsorbed, so as to achieve efficient film degradation.

Consideration

As a reason why the surfactant acted on the film to increase the amount of the enzyme adsorbed thereon, it was considered that the hydrophobic region of the surfactant faced towards the film, whereas the hydrophilic region thereof faced towards the side of the solution, so that the surface of the film became anionic and thus, the cationic region of the enzyme would be adsorbed on the film according to electrostatic interaction. In particular, when the anionic SDS was compared with the cationic DTMA-Cl, the amount of the enzyme adsorbed was largely changed only with the electric charge in the hydrophilic portion. Accordingly, it is highly likely that the electric charge in the hydrophilic region of the surfactant would be associated with adsorption of the enzyme. Thus, it is considered that, since the surfactant acted so that it mediated adsorption of the enzyme on the film, the improvement of the activity could not be confirmed in the activity measurement using a water-soluble substrate. Moreover, also as a reason why the enzyme has activity in a high concentration, it was considered that the adsorption was converted to adsorption according to the above-described electrostatic interaction, and thus it was not influenced by the distance between molecules.

[Example 3] Improvement of PET-hydrolyzing activity by using surfactant (2)

Whether sodium alkyl sulfate and sodium alkane sulfonate that are anionic surfactants improve the PET-hydrolyzing activity of PETase was confirmed.

As surfactants, sodium dodecyl sulfate (C12), sodium tridecyl sulfate (C13), sodium tetradecyl sulfate (C14), sodium dodecane-1-sulfonate (C12), sodium tridecane-1-sulfonate (C13) and sodium tetradecane-1-sulfonate (C14) were used.

As a surfactant, 0.01 to 0.04 (w/v)% of sodium dodecyl sulfate (C12), 0.005 to 0.0125 (w/v)% of sodium tridecyl sulfate (C13), 0.004 to 0.0075 (w/v)% of sodium tetradecyl sulfate (C14), 0.05 to 0.2 (w/v)% of sodium dodecane-1-sulfonate (C12), 0.04 to 0.07 (w/v)% of sodium tridecane-1-sulfonate (C13), or 0.015 to 0.03 (w/v)% of sodium tetradecane-1-sulfonate (C14) was used, and a buffer and a film were added thereto. The obtained mixture was pre-incubated at 30°C for 1 hour. Thereafter, the concentration of the enzyme was set at 50 nM, the film area was set at 3.39 cm$^2$/mL and the reaction was carried out for 3 hours (reaction scale: 500 μL).

A reaction system, to which no surfactants were added, was used as a control, and the aforementioned reaction system was compared with the control in terms of the amount of a degradation product, and the activity of the enzyme was indicated as a decomposed amount (%) compared with the control.

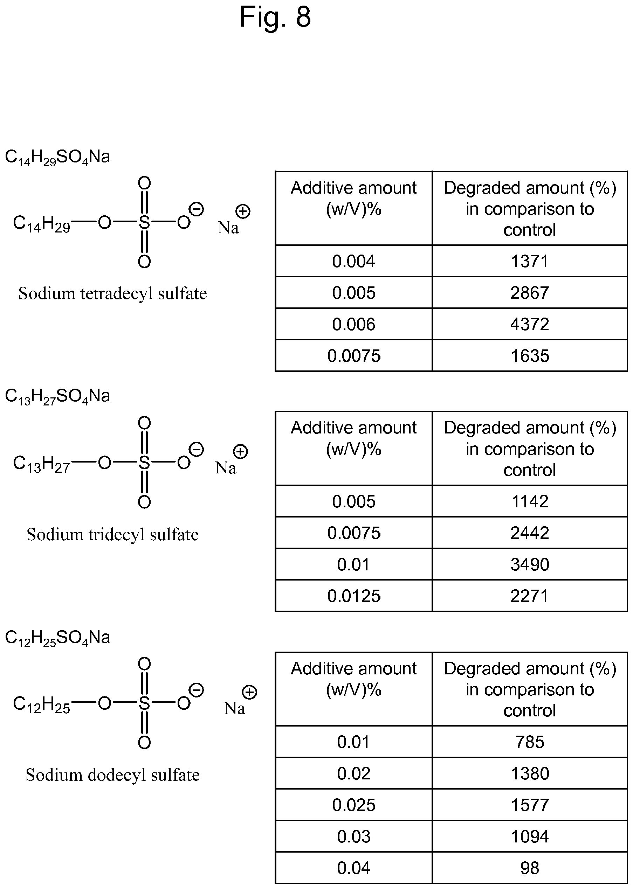

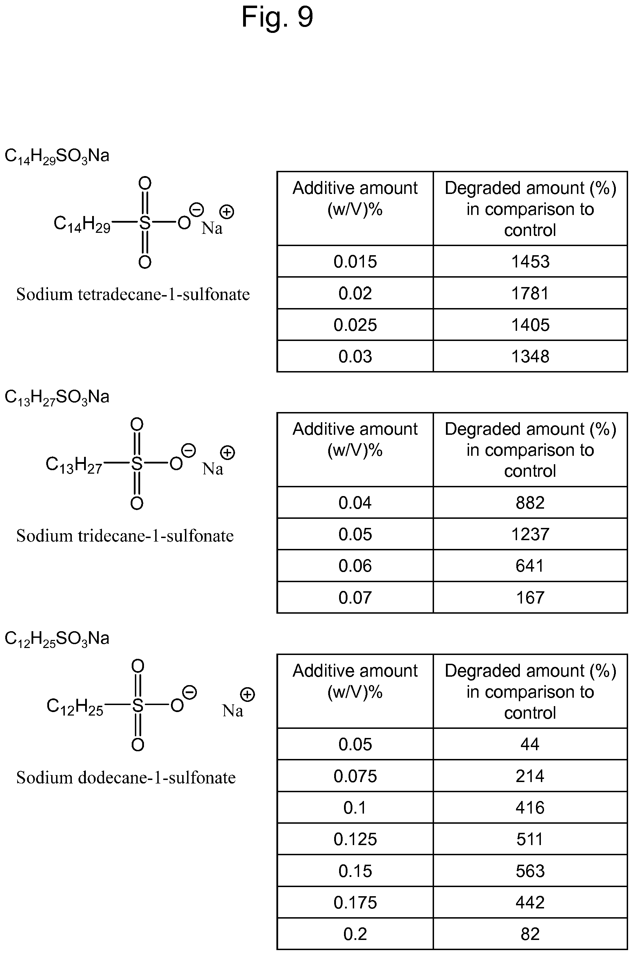

The results are shown in Figure 8 (sodium alkyl sulfate) and Figure 9 (sodium alkane sulfonate).

As shown in the figures, the improvement of the activity of PETase was observed, regardless of the type of the surfactant used.

Industrial Applicability

The method for improving the activity of PETase of the present invention can contribute to the effective utilization of PET resins.

All publications, patents and patent applications cited in the present description are incorporated herein by reference in their entirety.

## Claims

1. A method for improving PETase activity, comprising adding a surfactant, when the following PETase (a) or (b) is allowed to act on PET so as to degrade the PET:

   (a) an aromatic polyester-degrading enzyme consisting of the amino acid sequence as set forth in SEQ ID NO: 2 or 4 in the sequence listing; or
   (b) an aromatic polyester-degrading enzyme consisting of an amino acid sequence comprising a deletion, substitution or addition of one or several amino acids with respect to the amino acid sequence as set forth in SEQ ID NO: 2 or 4 in the sequence listing, and having an aromatic polyester-degrading activity.

2. The method according to claim 1, wherein the surfactant is selected from the group consisting of alkyl sulfate, alkane sulfonate, and polyoxyethylene alkyl phenyl ether.

3. The method according to claim 2, wherein the surfactant is sodium alkyl sulfate having alkyl containing 5 to 30 carbon atoms, or sodium alkane sulfonate having alkane containing 5 to 30 carbon atoms.

4. The method according to claim 2, wherein the surfactant is polyoxyethylene octyl phenyl ether or polyoxyethylene nonyl phenyl ether.

5. The method according to any one of claims 1 to 4, wherein the surfactant is added to the PET, and after a predetermined period of time, the PETase is added thereto.

6. The method according to any one of claims 1 to 4, wherein the surfactant and the PETase are simultaneously added to the PET.

7. A reagent composition kit for use in PET degradation, which comprises the following PETase (a) or (b) and a surfactant and improves PETase activity:

   (a) an aromatic polyester-degrading enzyme consisting of the amino acid sequence as set forth in SEQ ID NO: 2 or 4 in the sequence listing; or
   (b) an aromatic polyester-degrading enzyme consisting of an amino acid sequence comprising a deletion, substitution or addition of one or several amino acids with respect to the amino acid sequence as set forth in SEQ ID NO: 2 or 4 in the sequence listing, and having an aromatic polyester-degrading activity.

8.   The reagent composition kit according to claim 7, wherein the surfactant is selected from the group consisting of alkyl sulfate, alkane sulfonate, and polyoxyethylene alkyl phenyl ether.

9.   The reagent composition kit according to claim 8, wherein the surfactant is sodium alkyl sulfate having alkyl containing 5 to 30 carbon atoms, or sodium alkane sulfonate having alkane containing 5 to 30 carbon atoms.

10.   The reagent composition kit according to claim 8, wherein the surfactant is polyoxyethylene octyl phenyl ether or polyoxyethylene nonyl phenyl ether.

11.   The reagent composition kit according to any one of claims 7 to 10, comprising a mixture of the PETase and the surfactant.

12.   The reagent composition kit according to any one of claims 7 to 10, comprising the PETase and the surfactant, separately.

# Fig. 1

[A]

[B]

[C]

[D]

[E]

w+x+y+z=20

[F]

# Fig. 2

# Fig. 3

# Fig. 4

# Fig. 5

# Fig. 6

# Fig. 7

Lane 1: aqueous solution   Lane 6: not added
Lane 2: 0.01% SDS          Lane 7: 0.01% DTMA-Cl
Lane 3: 0.02% SDS          Lane 8: 0.02% DTMA-Cl
Lane 4: 0.03% SDS          Lane 9: 0.03% DTMA-Cl
Lane 5: 0.04% SDS          Lane 10: 0.04% DTMA-Cl

# Fig. 8

$C_{14}H_{29}SO_4Na$

Sodium tetradecyl sulfate

| Additive amount (w/V)% | Degraded amount (%) in comparison to control |
|---|---|
| 0.004 | 1371 |
| 0.005 | 2867 |
| 0.006 | 4372 |
| 0.0075 | 1635 |

$C_{13}H_{27}SO_4Na$

Sodium tridecyl sulfate

| Additive amount (w/V)% | Degraded amount (%) in comparison to control |
|---|---|
| 0.005 | 1142 |
| 0.0075 | 2442 |
| 0.01 | 3490 |
| 0.0125 | 2271 |

$C_{12}H_{25}SO_4Na$

Sodium dodecyl sulfate

| Additive amount (w/V)% | Degraded amount (%) in comparison to control |
|---|---|
| 0.01 | 785 |
| 0.02 | 1380 |
| 0.025 | 1577 |
| 0.03 | 1094 |
| 0.04 | 98 |

# Fig. 9

$C_{14}H_{29}SO_3Na$

$C_{14}H_{29}$—S—$O^{\ominus}$ $Na^{\oplus}$

Sodium tetradecane-1-sulfonate

| Additive amount (w/V)% | Degraded amount (%) in comparison to control |
|---|---|
| 0.015 | 1453 |
| 0.02 | 1781 |
| 0.025 | 1405 |
| 0.03 | 1348 |

$C_{13}H_{27}SO_3Na$

$C_{13}H_{27}$—S—$O^{\ominus}$ $Na^{\oplus}$

Sodium tridecane-1-sulfonate

| Additive amount (w/V)% | Degraded amount (%) in comparison to control |
|---|---|
| 0.04 | 882 |
| 0.05 | 1237 |
| 0.06 | 641 |
| 0.07 | 167 |

$C_{12}H_{25}SO_3Na$

$C_{12}H_{25}$—S—$O^{\ominus}$ $Na^{\oplus}$

Sodium dodecane-1-sulfonate

| Additive amount (w/V)% | Degraded amount (%) in comparison to control |
|---|---|
| 0.05 | 44 |
| 0.075 | 214 |
| 0.1 | 416 |
| 0.125 | 511 |
| 0.15 | 563 |
| 0.175 | 442 |
| 0.2 | 82 |

## INTERNATIONAL SEARCH REPORT

International application No.

PCT/JP2018/009170

A. CLASSIFICATION OF SUBJECT MATTER

Int.Cl.   C12N15/56(2006.01)i, C08J11/18(2006.01)i, C12N9/16(2006.01)i,
          C12P7/04(2006.01)i, C12P7/44(2006.01)i

According to International Patent Classification (IPC) or to both national classification and IPC

B. FIELDS SEARCHED

Minimum documentation searched (classification system followed by classification symbols)
Int.Cl.   C12N15/56, C08J11/18, C12N9/16, C12P7/04, C12P7/44

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JST7580 (JDreamIII), CAplus/BIOSIS (STN), UniProt/GeneSeq, DWPI (Thomson Innovation)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2015/025861 A1 (KEIO GIJUKU) 26 February 2015, claims (Family: none) | 1, 5-7, 11-12<br>2-4, 8-10 |
| Y<br>A | WO 2016/062695 A1 (CARBIOS) 28 April 2016, claims, pp. 9 1.29-10 1.13, 27 1.27-28 1.6 & US 2017/0313998 A1 & EP 3209771 A1 & JP 2018-500003 A & CN 106852154 A | 1, 5-7, 11-12<br>2-4, 8-10 |
| Y<br>A | JP 2003-510083 A (GESELLSCHAFT FÜR BIOTECHNOLOGISCHE FORSCHUNG MBH) 18 March 2003, claims & WO 2001/023581 A1, claims & US 2003/0194790 A1 & EP 1218519 A1 & DE 10030529 A & CA 2386013 A & KR 10-2002-0042694 A | 1, 5-7, 11-12<br>2-4, 8-10 |
| Y<br>A | JP 2006-124677 A (MITSUBISHI CHEMICAL CORP.) 18 May 2006, claims, paragraph [0040] (Family: none) | 1, 5-7, 11-12<br>2-4, 8-10 |

☐ Further documents are listed in the continuation of Box C.     ☐ See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 30 May 2018 (30.05.2018) | 12 June 2018 (12.06.2018) |

| Name and mailing address of the ISA/<br>   Japan Patent Office<br>   3-4-3, Kasumigaseki, Chiyoda-ku,<br>   Tokyo 100-8915, Japan | Authorized officer<br><br><br>Telephone No. |
|---|---|

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008199957 A
- WO 2015025861 A

- JP 2017049198 A

**Non-patent literature cited in the description**

- **YOSHIDA et al.** *Science,* 2016, vol. 351 (6278), 1196-1199

SEQUENCE LISTING

<110> KEIO UNIVERSITY

<120> A method of enhancing the activity of PET – degrading enzyme by an additive

<130> PH-7245-PCT

<150> JP 2017-049198
<151> 2017-03-14

<160> 4

<170> PatentIn version 3.5

<210> 1
<211> 873
<212> DNA
<213> Ideonella sp.

<220>
<221> CDS
<222> (1)..(873)

<400> 1

```
atg aac ttt ccc cgc gct tcc cgc ctg atg cag gcc gcc gtt ctc ggc      48
Met Asn Phe Pro Arg Ala Ser Arg Leu Met Gln Ala Ala Val Leu Gly
1               5                   10                  15

ggg ctg atg gcc gtg tcg gcc gcc gcc acc gcc cag acc aac ccc tac      96
Gly Leu Met Ala Val Ser Ala Ala Ala Thr Ala Gln Thr Asn Pro Tyr
            20                  25                  30

gcc cgc ggc ccg aac ccg aca gcc gcc tca ctc gaa gcc agc gcc ggc     144
Ala Arg Gly Pro Asn Pro Thr Ala Ala Ser Leu Glu Ala Ser Ala Gly
        35                  40                  45

ccg ttc acc gtg cgc tcg ttc acc gtg agc cgc ccg agc ggc tac ggc     192
Pro Phe Thr Val Arg Ser Phe Thr Val Ser Arg Pro Ser Gly Tyr Gly
    50                  55                  60

gcc ggc acc gtg tac tac ccc acc aac gcc ggc ggc acc gtg ggc gcc     240
Ala Gly Thr Val Tyr Tyr Pro Thr Asn Ala Gly Gly Thr Val Gly Ala
65                  70                  75                  80

atc gcc atc gtg ccg ggc tac acc gcg cgc cag tcg agc atc aaa tgg     288
Ile Ala Ile Val Pro Gly Tyr Thr Ala Arg Gln Ser Ser Ile Lys Trp
                85                  90                  95

tgg ggc ccg cgc ctg gcc tcg cac ggc ttc gtg gtc atc acc atc gac     336
Trp Gly Pro Arg Leu Ala Ser His Gly Phe Val Val Ile Thr Ile Asp
            100                 105                 110

acc aac tcc acg ctc gac cag ccg tcc agc cgc tcg tcg cag cag atg     384
Thr Asn Ser Thr Leu Asp Gln Pro Ser Ser Arg Ser Ser Gln Gln Met
        115                 120                 125

gcc gcg ctg cgc cag gtg gcc tcg ctc aac ggc acc agc agc agc ccg     432
Ala Ala Leu Arg Gln Val Ala Ser Leu Asn Gly Thr Ser Ser Ser Pro
    130                 135                 140
```

```
atc tac ggc aag gtc gac acc gcc cgc atg ggc gtg atg ggc tgg tcg        480
Ile Tyr Gly Lys Val Asp Thr Ala Arg Met Gly Val Met Gly Trp Ser
145                 150                 155                 160

atg ggc ggt ggc ggc tcg ctg atc tcg gcg gcc aac aac ccg tcg ctg        528
Met Gly Gly Gly Gly Ser Leu Ile Ser Ala Ala Asn Asn Pro Ser Leu
                    165                 170                 175

aaa gcc gcg gcg ccg cag gcc ccg tgg gac agc tcg acc aac ttc tcg        576
Lys Ala Ala Ala Pro Gln Ala Pro Trp Asp Ser Ser Thr Asn Phe Ser
                180                 185                 190

tcg gtc acc gtg ccc acg ctg atc ttc gcc tgc gag aac gac agc atc        624
Ser Val Thr Val Pro Thr Leu Ile Phe Ala Cys Glu Asn Asp Ser Ile
            195                 200                 205

gcc ccg gtc aac tcg tcc gcc ctg ccg atc tac gac agc atg tcg cgc        672
Ala Pro Val Asn Ser Ser Ala Leu Pro Ile Tyr Asp Ser Met Ser Arg
        210                 215                 220

aat gcg aag cag ttc ctc gag atc aac ggt ggc tcg cac tcc tgc gcc        720
Asn Ala Lys Gln Phe Leu Glu Ile Asn Gly Gly Ser His Ser Cys Ala
225                 230                 235                 240

aac agc ggc aac agc aac cag gcg ctg atc ggc aag aag ggc gtg gcc        768
Asn Ser Gly Asn Ser Asn Gln Ala Leu Ile Gly Lys Lys Gly Val Ala
                    245                 250                 255

tgg atg aag cgc ttc atg gac aac gac acg cgc tac tcc acc ttc gcc        816
Trp Met Lys Arg Phe Met Asp Asn Asp Thr Arg Tyr Ser Thr Phe Ala
                260                 265                 270

tgc gag aac ccg aac agc acc cgc gtg tcg gac ttc cgc acc gcg aac        864
Cys Glu Asn Pro Asn Ser Thr Arg Val Ser Asp Phe Arg Thr Ala Asn
                275                 280                 285

tgc agc tga                                                            873
Cys Ser
290
```

<210> 2
<211> 290
<212> PRT
<213> Ideonella sp.

<400> 2

```
Met Asn Phe Pro Arg Ala Ser Arg Leu Met Gln Ala Ala Val Leu Gly
1               5                   10                  15

Gly Leu Met Ala Val Ser Ala Ala Ala Thr Ala Gln Thr Asn Pro Tyr
            20                  25                  30

Ala Arg Gly Pro Asn Pro Thr Ala Ala Ser Leu Glu Ala Ser Ala Gly
        35                  40                  45

Pro Phe Thr Val Arg Ser Phe Thr Val Ser Arg Pro Ser Gly Tyr Gly
        50                  55                  60
```

```
Ala Gly Thr Val Tyr Tyr Pro Thr Asn Ala Gly Gly Thr Val Gly Ala
65                  70              75                  80

Ile Ala Ile Val Pro Gly Tyr Thr Ala Arg Gln Ser Ser Ile Lys Trp
                85              90                  95

Trp Gly Pro Arg Leu Ala Ser His Gly Phe Val Val Ile Thr Ile Asp
            100             105             110

Thr Asn Ser Thr Leu Asp Gln Pro Ser Ser Arg Ser Ser Gln Gln Met
        115             120             125

Ala Ala Leu Arg Gln Val Ala Ser Leu Asn Gly Thr Ser Ser Ser Pro
        130             135             140

Ile Tyr Gly Lys Val Asp Thr Ala Arg Met Gly Val Met Gly Trp Ser
145             150             155             160

Met Gly Gly Gly Gly Ser Leu Ile Ser Ala Ala Asn Asn Pro Ser Leu
            165             170             175

Lys Ala Ala Ala Pro Gln Ala Pro Trp Asp Ser Ser Thr Asn Phe Ser
        180             185             190

Ser Val Thr Val Pro Thr Leu Ile Phe Ala Cys Glu Asn Asp Ser Ile
        195             200             205

Ala Pro Val Asn Ser Ser Ala Leu Pro Ile Tyr Asp Ser Met Ser Arg
        210             215             220

Asn Ala Lys Gln Phe Leu Glu Ile Asn Gly Gly Ser His Ser Cys Ala
225             230             235             240

Asn Ser Gly Asn Ser Asn Gln Ala Leu Ile Gly Lys Lys Gly Val Ala
            245             250             255

Trp Met Lys Arg Phe Met Asp Asn Asp Thr Arg Tyr Ser Thr Phe Ala
        260             265             270

Cys Glu Asn Pro Asn Ser Thr Arg Val Ser Asp Phe Arg Thr Ala Asn
        275             280             285

Cys Ser
290
```

<210> 3
<211> 792

<212>  DNA
<213>  Ideonella sp.


<220>
<221>  CDS
<222>  (1)..(792)

<400>  3

```
caa aca aac ccg tat gcg cgt ggt ccg aat ccg act gct gcc agc ctt      48
Gln Thr Asn Pro Tyr Ala Arg Gly Pro Asn Pro Thr Ala Ala Ser Leu
1               5                   10                  15

gaa gcc tct gct ggc cct ttc acc gta cgc tcg ttc acg gtt tcg cgt      96
Glu Ala Ser Ala Gly Pro Phe Thr Val Arg Ser Phe Thr Val Ser Arg
                20                  25                  30

cca tcg ggc tat ggt gca ggc acc gtg tat tac ccg aca aat gct ggc     144
Pro Ser Gly Tyr Gly Ala Gly Thr Val Tyr Tyr Pro Thr Asn Ala Gly
            35                  40                  45

ggg act gta ggt gcc att gcg att gtt ccg ggc tat acg gct cgt cag     192
Gly Thr Val Gly Ala Ile Ala Ile Val Pro Gly Tyr Thr Ala Arg Gln
        50                  55                  60

tca agc atc aaa tgg tgg ggt cca cgt ctg gca agc cat ggc ttt gtg     240
Ser Ser Ile Lys Trp Trp Gly Pro Arg Leu Ala Ser His Gly Phe Val
65                  70                  75                  80

gtc atc acc att gac acg aat tct acg ctg gat cag ccg agc agt cgg     288
Val Ile Thr Ile Asp Thr Asn Ser Thr Leu Asp Gln Pro Ser Ser Arg
                85                  90                  95

agc tca cag cag atg gcc gcc tta cgc caa gtt gca tcg tta aac gga     336
Ser Ser Gln Gln Met Ala Ala Leu Arg Gln Val Ala Ser Leu Asn Gly
            100                 105                 110

aca tcc tca tcg cca atc tac ggg aaa gtg gat act gcc cgc atg gga     384
Thr Ser Ser Ser Pro Ile Tyr Gly Lys Val Asp Thr Ala Arg Met Gly
            115                 120                 125

gtg atg ggc tgg agt atg ggt ggt ggt ggc agt ctc att tcc gcg gcg     432
Val Met Gly Trp Ser Met Gly Gly Gly Gly Ser Leu Ile Ser Ala Ala
        130                 135                 140

aac aat ccc tct ctg aaa gcg gca gcg ccg caa gcg ccc tgg gat tca     480
Asn Asn Pro Ser Leu Lys Ala Ala Ala Pro Gln Ala Pro Trp Asp Ser
145                 150                 155                 160

agc acc aac ttt tcc agt gtt acc gtc ccg acc ttg atc ttt gcg tgc     528
Ser Thr Asn Phe Ser Ser Val Thr Val Pro Thr Leu Ile Phe Ala Cys
                165                 170                 175

gaa aac gac agc att gca cct gtg aac agc tct gct ctg cct att tac     576
Glu Asn Asp Ser Ile Ala Pro Val Asn Ser Ser Ala Leu Pro Ile Tyr
            180                 185                 190

gat agc atg tcc cgc aat gca aag cag ttc ctg gag atc aac ggt ggg     624
Asp Ser Met Ser Arg Asn Ala Lys Gln Phe Leu Glu Ile Asn Gly Gly
            195                 200                 205

tca cac tcg tgt gcc aat tcc ggc aat agc aat cag gcg ctg att ggc     672
Ser His Ser Cys Ala Asn Ser Gly Asn Ser Asn Gln Ala Leu Ile Gly
```

```
                210                      215                      220

          aag aaa gga gtg gcc tgg atg aaa cgc ttc atg gat aac gat acc cgc         720
          Lys Lys Gly Val Ala Trp Met Lys Arg Phe Met Asp Asn Asp Thr Arg
          225                     230                     235                     240

          tat tcc acc ttt gcg tgt gaa aac ccg aat agt acc cgt gtc agt gac         768
          Tyr Ser Thr Phe Ala Cys Glu Asn Pro Asn Ser Thr Arg Val Ser Asp
                              245                     250                     255

          ttt cgc acg gcg aac tgc tct taa                                         792
          Phe Arg Thr Ala Asn Cys Ser
                          260


          <210>  4
          <211>  263
          <212>  PRT
          <213>  Ideonella sp.

          <400>  4

          Gln Thr Asn Pro Tyr Ala Arg Gly Pro Asn Pro Thr Ala Ala Ser Leu
          1               5                   10                  15


          Glu Ala Ser Ala Gly Pro Phe Thr Val Arg Ser Phe Thr Val Ser Arg
                          20                  25                  30


          Pro Ser Gly Tyr Gly Ala Gly Thr Val Tyr Tyr Pro Thr Asn Ala Gly
                  35                  40                  45


          Gly Thr Val Gly Ala Ile Ala Ile Val Pro Gly Tyr Thr Ala Arg Gln
                  50                  55                  60


          Ser Ser Ile Lys Trp Trp Gly Pro Arg Leu Ala Ser His Gly Phe Val
          65                  70                  75                  80


          Val Ile Thr Ile Asp Thr Asn Ser Thr Leu Asp Gln Pro Ser Ser Arg
                              85                  90                  95


          Ser Ser Gln Gln Met Ala Ala Leu Arg Gln Val Ala Ser Leu Asn Gly
                          100                 105                 110


          Thr Ser Ser Ser Pro Ile Tyr Gly Lys Val Asp Thr Ala Arg Met Gly
                          115                 120                 125


          Val Met Gly Trp Ser Met Gly Gly Gly Gly Ser Leu Ile Ser Ala Ala
                  130                 135                 140


          Asn Asn Pro Ser Leu Lys Ala Ala Ala Pro Gln Ala Pro Trp Asp Ser
          145                 150                 155                 160


          Ser Thr Asn Phe Ser Ser Val Thr Val Pro Thr Leu Ile Phe Ala Cys
```

16

165       170       175

Glu Asn Asp Ser Ile Ala Pro Val Asn Ser Ser Ala Leu Pro Ile Tyr
180       185       190

Asp Ser Met Ser Arg Asn Ala Lys Gln Phe Leu Glu Ile Asn Gly Gly
195       200       205

Ser His Ser Cys Ala Asn Ser Gly Asn Ser Asn Gln Ala Leu Ile Gly
210       215       220

Lys Lys Gly Val Ala Trp Met Lys Arg Phe Met Asp Asn Asp Thr Arg
225       230       235       240

Tyr Ser Thr Phe Ala Cys Glu Asn Pro Asn Ser Thr Arg Val Ser Asp
245       250       255

Phe Arg Thr Ala Asn Cys Ser
260